# EUROPEAN PATENT APPLICATION

(11) **EP 1 041 148 A1**
(43) Date of publication of application: **04.10.2000**
(21) Application number: 99201065.2
(22) Date of filing: 02.04.1999
(51) Int. Cl.: C12N 15/29, C12N 15/82, A01N 65/00

(54) **Pathogen inducible promoter**

(71) Applicant: MOGEN INTERNATIONAL N.V., NL-2333 CB Leiden (NL)
(72) Inventor: The designation of the inventor has not yet been filed
(74) Representative: van Wezenbeek, Lambertus A.C.M.

(57) **Abstract**

This invention describes a pathogen-inducible promoter isolated from *Arabidopsis thaliana.* Also claimed are chimeric constructs where this pathogen-inducible promoter drives expression of antipathogenic proteins or of proteins which can elicit a hypersensitive response.

SEQUENCE LISTING

<110> Mogen International nv

<120> Pathogen Inducible Promoter

<130> pathogen inducible promoter

<140>

<141>

<160> 9

<170> PatentIn Ver. 2.0

<210> 1

<211> 563

<212> DNA

<213> Arabidopsis thaliana

<400> 1

ccatggtccg tcctgtagaa accccaaccc gtgaaatcaa aaaactcgac ggcctgtggg 60

cattcagtct ggatcgcgaa aactgtggaa ttgatcagcg ttggtgggaa agcgcgttac 120

aagaaagccg ggcaattgct gtgccaggca gttttaacga tcagttcgcc gatgcagata 180

ttcgtaatta tgcgggcaac gtctggtatc agcgcgaagt ctttataccg aaaggttggg 240

caggccagcg tatcgtgctg cgtttcgatg cggtcactca ttacggcaaa gtgtgggtca 300

ataatcagga agtgatggag catcagggcg gctatacgcc atttgaagcc gatgtcacgc 360

cgtatgttat tgccgggaaa agtgtacgta tcaccgtttg tgtgaacaac gaactgaact 420

ggcagactat cccgccggga atggtgatta ccgacgaaaa cggcaagaaa aagcagtctt 480

acttccatga tttctttaac tatgccggaa tccatcgcag cgtaatgctc tacaccacgc 540

cgaacacctg ggtggacgat atc 563

<210> 2

<211> 20

<212> DNA

<213> Unknown

<220>

<223> Description of Unknown Organism: synthetic
sequence

<400> 2

ctttcccacc aacgctgatc 20

<210> 3

<211> 21

<212> DNA

<213> Unknown

<220>

<223> Description of Unknown Organism:synthetic sequence

<400> 3

cgcaccatcg tcggctacag c 21

<210> 4

<211> 21

<212> DNA

<213> Unknown

<220>

<223> Description of Unknown Organism:synthetic sequence

<400> 4

gtaatgctct acaccacgcc g 21

<210> 5

<211> 401

<212> DNA

<213> Arabidopsis sp.

<400> 5

gttgacaaaa aaagtaaaat aaagttctaa aatgcttttt gaccaatttt caataagaaa 60

tagtaatgtt ataaaattcg tagtaataaa agtatttata aaaaaaaaaa gtataaacac 120

cgcaattttc ggtcggagtt cgtggcatgt aactacttac tttactgtca cttaaaggtc 180

tgatttcttc gttgtctcta aacgtgaaga cgatcaccga atccaaatcc tcacaacgac 240

atgttcttca agatatttga ctctcttata aacctaacga gacaagttta tgaatatgtt 300

cctttgttta cacttgtaag aaaaccgaaa cccatttaca tgtctctgtt tcgtcccttc 360

atatagtata aaagattcaa atatatatct ggagaaagct t 401

<210> 6

<211> 2348

<212> DNA

<213> Arabidopsis thaliana

<400> 6

gatatcatca aataatacaa attccatgtt ctaattagca cacaagatta agatgaagtg 60

acttgatatt tggtcaacca ttctcgaatt ctacagaatc tctagtaact tgtaagccaa 120

aggcagaaac aaaatttaaa accctaaaaa taatggtaaa gaccatcaat agaatatacc 180

ttcagcttct ctttgtccaa gacgatcact tccgttgcac atatctagaa gtccgaagca 240

attataacgc atcacaaaca cagacaaaaa ttcctcccaa ggaacagagg aaaaaagctc 300

aaattcaaat tcacaagcaa aataatattc agaaaagggt ttgatagaga tatgaaatgg 360

aacttttctt taaaagaacc tcaatttcga cctgtctcct cgatactaaa tctataaagt 420

aacgatgaga gaagaagaag aagaagataa tgtgtttatt tgctagtgcg aagaagaaat 480

ctatattgag ttaatcaaag agaaatcatg agactgcttt tgtctgattt aaaatttaaa 540

ctttttcgtg aagatgtcaa taaattgttg agcatacaaa gcaaaccaaa cccagaactc 600

atgaacagtg aatacgctaa aaggtattca attttatttt gttgtttatg ttcggctgct 660

gatgctcgaa agtgacaaac gaaagtgaca gctacacgac gacgagactt ttattactat 720

attttatttt acttttttca cacgacgaga ctattataaa agtcgcactg attagatttc 780

ttctttaatt attttcttct gcctgtaact aaggcaaggt tagaagaatt caacttcaat 840

ggattttata gacctgttta ttacaattta agtcacacaa atacgccaaa caaattctat 900

cacttaccta cctttttttt acattttaca ataattgtcc tcgtaccgtt taactaagaa 960
aggttctcat agactcatac gtacatacgt atgaattaag agcgatagtt actaataaag 1020
ttttgtttac ctgaccaaac tacacggcac atttaatttg accaagttct tctcgccgtt 1080
cagacaacca tgcatttata tttttctctt aaaaaagaaa tttttgccaa cattttataa 1140
cgtcatcaaa cgagctttta cttgacgtca tcactacgct gacattcaat catccaaaga 1200
gaaagttcga attctcaaat cccaactcaa tgatccaaaa aggaattttt gttttcacat 1260
tgccttgcgt ctttctttct ttcttaaaac gaatcgtttg ttaatttcta tcgattgcgg 1320
tctgaaattg aatttacagt tggcacatga catcatcgta ccacgaaaat cctccattat 1380
ttcttatttg cgccacaaaa tcaaaacttg aaaagtccac cttgttgaca aaaaaagtaa 1440
aataaagttc taaaatgttt ttgaccaatt ttcaataaga aatagtaatc gttataaaat 1500
tgtagtaata aaagtattta taaaaaaaaa aagtataaac acgattttgg tggagttgtg 1560
gcatgtaact acttacttta ctcgtcactt aaaggtctga tttcttgttg tctctaaacg 1620
tgaagacgat tcaccgaatc caaatcctca caacgacatg ttcttcaagt atatttgact 1680
ctcttataaa cctaacgaga caagtttatg aatatgttcc tttgtttaca cttgtaagaa 1740
aaccgaaacc catttacatg tctctgtttc gtcccttcat atagtataaa agattcaaat 1800
atatatctgg agattcttat cgtacaactt ttttttttgt atcggtcata ttggaatgaa 1860
ccattttggt acatgccaaa agctgtaaat tttcaagtaa tttagtggat ttacaaagag 1920
ggcttgtccc gcgagattta ggtaccaaac tcgttaaatt cttcttctat gactctgttt 1980
tgatcttctt tactatttcc atgttttgtt ctgtttctcg attctcactt tgttctatat 2040
cttggtttaa aatatcagac acagcattta gcaattgctt tgggagaaca ttatgaagga 2100
aaaagacatt tgatgcctga ggtttataga ttatacggtt gatcacttag aaacaagttg 2160
cttcgaagcg tgagaacaaa aaacaagaac acagattaca taataatatg ggtcaagatg 2220
tgaacttggg gtactggttc cgcggagaag cagcatagct tctttcacta agaagatcat 2280
cattttcggc ttcgtttccg tctaagcaca gaatgttgat gttgtcaaca ctcgggtcag 2340

atgagctc 2348

<210> 7

<211> 1782

<212> DNA

<213> Arabidopsis thaliana

<400> 7

ctcgaggtca accattctcg aattctacag aatctctagt aacttgtaag ccaaaggcag 60

aaacaaaatt taaaacccta aaaataatgg taaagaccat caatagaata taccttcagc 120

ttctctttgt ccaagacgat cacttccgtt gcacatatct agaagtccga agcaattata 180

acgcatcaca aacacagaca aaaattcctc ccaaggaaca gaggaaaaaa gctcaaattc 240

aaattcacaa gcaaaataat attcagaaaa gggtttgata gagatatgaa atggaacttt 300

tctttaaaag aacctcaatt tcgacctgtc tcctcgatac taaatctata aagtaacgat 360

gagagaagaa gaagaagaag ataatgtgtt tatttgctag tgcgaagaag aaatctatat 420

tgagttaatc aaagagaaat catgagactg cttttgtctg atttaaaatt taaacttttt 480

cgtgaagatg tcaataaatt gttgagcata caaagcaaac caaacccaga actcatgaac 540

agtgaatacg ctaaaaggta ttcaatttta ttttgttgtt tatgttcggc tgctgatgct 600

cgaaagtgac aaacgaaagt gacagctaca cgacgacgag acttttatta ctatatttta 660

ttttactttt ttcacacgac gagactatta taaaagtcgc actgattaga tttcttcttt 720

aattattttc ttctgcctgt aactaaggca aggttagaag aattcaactt caatggattt 780

tatagacctg tttattacaa tttaagtcac acaaatacgc caaacaaatt ctatcactta 840

cctacctttt ttttacattt tacaataatt gtcctcgtac cgtttaacta agaaaggttc 900

tcatagactc atacgtacat acgtatgaat taagagcgat agttactaat aaagttttgt 960

ttacctgacc aaactacacg gcacatttaa tttgaccaag ttcttctcgc cgttcagaca 1020

accatgcatt tatatttttc tcttaaaaaa gaaatttttg ccaacatttt ataacgtcat 1080

caaacgagct tttacttgac gtcatcacta cgctgacatt caatcatcca aagagaaagt 1140

tcgaattctc aaatcccaac tcaatgatcc aaaaaggaat ttttgttttc acattgcctt 1200

gcgtctttct ttctttctta aaacgaatcg tttgttaatt tctatcgatt gcggtctgaa 1260

attgaattta cagttggcac atgacatcat cgtaccacga aaatcctcca ttatttctta 1320

tttgcgccac aaaatcaaaa cttgaaaagt ccaccttgtt gacaaaaaaa gtaaaataaa 1380

gttctaaaat gtttttgacc aattttcaat aagaaatagt aatcgttata aaattgtagt 1440

aataaaagta tttataaaaa aaaaaagtat aaacacgatt ttggtggagt tgtggcatgt 1500

aactacttac tttactcgtc acttaaaggt ctgatttctt gttgtctcta aacgtgaaga 1560

cgattcaccg aatccaaatc ctcacaacga catgttcttc aagtatattt gactctctta 1620

taaacctaac gagacaagtt tatgaatatg ttcctttgtt tacacttgta agaaaaccga 1680

aacccattta catgtctctg tttcgtccct tcatatagta taaaagattc aaatatatat 1740

ctggagaaag cttggatccc cgggtaggtc agtcccccat gg 1782

<210> 8

<211> 20

<212> DNA

<213> Unknown

<220>

<223> Description of Unknown Organism:synthetic sequence

<400> 8

cgtaccatgg gggactgacc                                        20

<210> 9

<211> 37

<212> DNA

<213> Unknown

<220>

<223> Description of Unknown Organism:synthetic sequence

<400> 9

agccgagctc gttgacaaaa aaagtaaaat aaagttc                     37

## Description

### Field of the Invention

This invention relates to the field of pathogen inducible promoters and chimeric DNA sequences comprising said promoters, especially in the area of plant biotechnology.

### Background Art

Inducible promoters include any promoter capable of increasing the amount of gene product produced by a given gene, in response to exposure to an inducer. In the absence of an inducer the DNA sequence will not be transcribed. Typically, the factor that binds specifically to an inducible promoter to activate transcription is present in an inactive form which is then directly or indirectly converted to the active form by the inducer. The inducer may be a chemical agent such as a protein, metabolite (sugar, alcohol, etc.), a growth regulator, herbicide, or a phenolic compound or a physiological stress imposed directly by heat, salt, wounding, toxic elements etc., or indirectly through the action of a pathogen or disease agent such as a virus. A plant cell containing an inducible promoter may be exposed to an inducer by externally applying the inducer to the cell such as by spraying, watering, heating, or similar methods. Inducible promoters are known to those familiar with the art and several exist that could conceivably be used to drive expression of the genes of the invention. Examples of inducible promoters include the inducible 70 kD heat shock promoter of *Drosophila melanogaster* (Freeling, M. *et al.,* Ann. Rev. Genet. 19, 297-323) and the alcohol dehydrogenase promoter which is induced by ethanol (Nagao, R.T. *et al.,* in: Miflin, B.J. (ed.) Oxford Surveys of Plant Molecular and Cell Biology, Vol. 3., pp. 384-438, Oxford Univ. Press, 1986). Examples for promoters that are inducible by a simple chemical are the promoters described in WO 90/08826, WO 93/21334, WO 93/031294 and WO 96/37609.

An important subclass of inducible promoters are the promoters which are induced in plants upon pathogen infection. As examples of a pathogen-inducible promoter the PRP1 promoter (also named gstl promoter) obtainable from potato (Martini N. *et al.* (1993), Mol. Gen. Genet. 263, 179-186), the *Fis1* promoter (WO 96/34949), the *Bet ν 1* promoter (Swoboda, I., *et al.,* Plant, Cell and Env. 18, 865-874, 1995), the *Vst1* promoter (Fischer, R., Dissertation, Univ. of Hohenheim, 1994; Schubert, R., *et al.* Plant Mol. Biol. 34, 417-426, 1997), the sesquiterpene cyclase promoter (Yin, S., *et al.,* Plant Physiol. 115, 437-451, 1997) and the *gstA1* promoter (Mauch, F. and Dudler, R., Plant Physiol. 102, 1193-1201, 1993) may be mentioned. A drawback of some of these promoters is that they are also active constitutively or that they do not react to certain types of pathogens. Furthermore, it would be advantageous to have promoters that regulate expression very soon after pathogen infection, *i.e.* with as short as possible induction times.

Thus, there is still need for promoters that are pathogen-inducible which overcome the disadavantages of the prior art.

### Summary of the Invention

We now have found a DNA fragment obtainable from *Arabidopsis thaliana,* capable of promoting pathogen-inducible transcription of an associated DNA sequence when re-introduced into a plant. Said DNA fragment is more specifically characterized in that it comprises the nucleotide sequence from 1 to 1782 depicted in SEQ ID NO: 7.

Also included in the invention is a portion or variant of a DNA fragment according to that described above, capable of promoting pathogen-inducible transcription of an associated DNA sequence when re-introduced into a plant.

Embodiments of the invention are chimeric DNA sequences comprising in the direction of transcription a DNA fragment according to any one of the DNA fragments described above and a DNA sequence to be expressed under the transcriptional control thereof and which is not naturally under transcriptional control of said DNA fragment.

A preferred embodiment is such a chimeric DNA sequence wherein the DNA sequence to be expressed causes the production of an antipathogenic protein, which is preferably selected from the group consisting of chitinases, glucanases, osmotins, magainins, lectins, saccharide oxidase like hexose oxidases, oxalate oxidase, oxalate decarboxylase, toxins from *Bacillus thuringiensis,* antifungal proteins isolated from *Mirabilis jalapa, Amaranthus, Raphanus, Brassica, Sinapis, Arabidopsis, Dahlia, Cnicus, Lathyrus, Clitoria, Allium* seeds, *Aralia* and *Impatiens* and albumin-type proteins, such as thionine, napin, barley trypsin inhibitor, cereal gliadin and wheat-alpha-amylase.

Another embodiment of the chimeric DNA sequences of the invention is a chimeric DNA sequence wherein the DNA sequence to be expressed causes the production of a protein that can induce a hypersensitive response, preferably selected from the group consisting of CF and Pto proteins from tomato, avr proteins from *Cladosporium fulvum* and elicitor proteins from *Pseudomonas* or *Xanthomonas.*

Further part of the invention are replicons comprising above mentioned chimeric DNA sequences preferably having at least one recognition site for a restriction endonuclease for insertion of a DNA sequence to be expressed under the control of said DNA fragment.

Also included in the invention are microorganism containing such a replicon, plant cells having incorporated into their genome a chimeric DNA sequence according to those described above, and plants essentially consisting of said cells. Such a plant is preferably a dicotyledonous plant. Also part of said plants selected from seeds, flowers, tubers, roots, leaves, fruits, pollen and wood, form part of the invention.

Yet another embodiment of the invention is the use of a DNA fragment as described above for identifying homologues capable of promoting pathogen-induced transcription in a plant.

Further use of a chimeric DNA sequence according to the invention for transforming plants and use of a portion or variant of the DNA fragments according to the invention for making hybrid regulatory DNA sequences is part of the invention.

Another object of the invention is the use of a chimeric DNA sequence as described above for conferring pathogen resistance to a plant.

### Detailed Description of the Figures

The invention is illustrated with reference to the Examples and figures in which :
Figure 1 shows: a photograph of histochemical GUS staining of *Arabidopsis thaliana* line 533-488 leaf infected with *Botrytis cinerea*
Figure 2 shows: a Southern blot of genomic DNA of *Arabidopsis thaliana* line 533-488 hybridised with GUS probe
Figure 3 shows: a schematic map of plasmid pMOG1040
Figure 4 shows: a schematic map of plasmid pMOG1056
Figure 5 shows: a photograph of GUS stained *Brassica napus* leaf infected with *Phoma lingam*
Figure 6 shows: a photograph of GUS stained *Brassica napus* stem infected with *Phoma lingam*

### Detailed Description of the Invention

The main aspect of the invention are regulatory sequences naturally occurring in *Arabidopsis thaliana.* It has been found that upon pathogen infection genes under the regulatory control of the regulatory sequence are highly expressed, indicating pathogen inducibility. Pathogen inducible promoters are of great value in biotechnological resistance engineering.

In this description the terms 'regulatory sequence' and 'promoter' are used interchangeably.

The present invention provides amongst others chimeric DNA sequences which comprise the DNA fragments according to the invention. The expression chimeric DNA sequence shall mean to comprise any DNA sequence which comprises DNA sequences not naturally found in nature. For instance, chimeric DNA shall mean to comprise DNA comprising the regulatory region which is pathogen-inducible in a non-natural location of the plant genome, notwithstanding the fact that said plant genome normally contains a copy of the said regulatory region in its natural chromosomal location. Similarly, the said regulatory region may be incorporated in the plant genome wherein it is not naturally found, or in a replicon or vector where it is not naturally found, such as a bacterial plasmid or a viral vector. Chimeric DNA shall not be limited to DNA molecules which are replicable in a host, but shall also mean to comprise DNA capable of being ligated into a replicon, for instance by virtue of specific adaptor sequences, physically linked to the regulatory region according to the invention. The regulatory region may or may not be linked to its natural downstream open reading frame.

The open reading frame of the gene which expression is driven by the pathogen-inducible regulatory regions of the invention may be derived from a genomic library. In this latter it may contain one or more introns separating the exons making up the open reading frame that encodes a protein according to the invention. The open reading frame may also be encoded by one uninterrupted exon, or by a cDNA to the mRNA encoding a protein according to the invention. Chimeric DNA sequences according to the invention also comprise those in which one or more introns have been artificially removed or added. Each of these variants is embraced by the present invention.

In order to be capable of being expressed in a host cell a regulatory region according to the invention will usually be provided with a transcriptional initiation region which may be suitably derived from any gene capable of being expressed in the host cell of choice, as well as a translational initiation region for ribosome recognition and attachment. In eukaryotic cells, an expression cassette usually comprises in addition a transcriptional termination region located downstream of said open reading frame, allowing transcription to terminate and polyadenylation of the primary transcript to occur. In addition, the codon usage may be adapted to accepted codon usage of the host of choice. Further, often a signal sequence may be encoded, which is responsible for the targeting of the gene expression product to subcellular compartments. The principles governing the expression of a chimeric DNA construct in a chosen host cell are commonly understood by those of ordinary skill in the art and the construction of expressible chimeric DNA constructs is now routine for any sort of host cell, be it prokaryotic or eukaryotic.

In order for the chimeric DNA sequence to be maintained in a host cell it will usually be provided in the form of a replicon comprising said chimeric DNA sequence according to the invention linked to DNA which is recognised and replicated by the chosen host cell. Accordingly, the selection of the replicon is determined largely by the host cell of choice. Such principles as govern the selection of suitable replicons for a particular chosen host are well within the realm of the ordinary skilled person in the art.

A special type of replicon is one capable of transferring itself, or a part thereof, to another host cell, such as a plant cell, thereby co-transferring the open reading frame according to the invention to said plant cell. Replicons with such capability are herein referred to as vectors. An example of such vector is a Ti-plasmid vector which, when present in a suitable host, such as *Agrobacterium tumefaciens,* is capable of transferring part of itself, the so-called T-region, to a plant cell. Different types of Ti-plasmid vectors *(vide:* EP 0 116 718 B1) are now routinely being used to transfer chimeric DNA sequences into plant cells, or protoplasts, from which new plants may be generated which stably incorporate said chimeric DNA in their genomes. A particularly preferred form of Ti-plasmid vectors are the so-called binary vectors as claimed in (EP 0 120 516 B1 and US 4,940,838). Other suitable vectors, which may be used to introduce DNA according to the invention into a plant host, may be selected from the viral vectors, *e.g.* non-integrative plant viral vectors, such as derivable from the double stranded plant viruses (*e.g*. CaMV) and single stranded viruses, gemini viruses and the like. The use of such vectors may be advantageous, particularly when it is difficult to stably transform the plant host. Such may be the case with woody species, especially trees and vines.

The expression "host cells incorporating a chimeric DNA sequence according to the invention in their genome" shall mean to comprise cells, as well as multicellular organisms comprising such cells, or essentially consisting of such cells, which stably incorporate said chimeric DNA into their genome thereby maintaining the chimeric DNA, and preferably transmitting a copy of such chimeric DNA to progeny cells, be it through mitosis or meiosis. According to a preferred embodiment of the invention plants are provided, which essentially consist of cells which incorporate one or more copies of said chimeric DNA into their genome, and which are capable of transmitting a copy or copies to their progeny, preferably in a Mendelian fashion. By virtue of the transcription and translation of the chimeric DNA according to the invention in some or all of the plant's cells, those cells that comprise said regulatory region will respond to pathogen attack and thus produce the protein encoded by the open reading frame which is under control of the regulatory region. In specific embodiments of the invention this protein will be an antipathogenic protein which is capable of conferring resistance to pathogen infections.

As is well known to those of skill in the art, regulatory regions of plant genes consist of disctinct subregions with interesting properties in terms of gene expression. Examples of subregions as meant here, are enhancers but also silencers of transcription. These elements may work in a general (constitutive) way, or in a tissue-specific manner. Deletions may be made in the regulatory DNA sequences according to the invention, and the sub fragments may be tested for expression patterns of the associated DNA. Various subfragments so obtained, or even combinations thereof, may be useful in methods of engineering pathogen resistance, or other applications involving the expression of heterologous DNA in plants. The use of DNA sequences according to the invention to identify functional subregions, and the subsequent use thereof to promote or suppress gene expression in plants is also encompassed by the present invention.

As regards the necessity of a transcriptional terminator region, it is generally believed that such a region enhances the reliability as well as the efficiency of transcription in plant cells. Use thereof is therefore strongly preferred in the context of the present invention.

Examples of proteins that may be used in combination with the regulatory region according to the invention include, but are not limited to, β-1,3-glucanases and chitinases which are obtainable from barley (Swegle M. *et al.,* Plant Mol. Biol. 12, 403-412, 1989; Balance G.M. *et al.,* Can. J. Plant Sci. 56, 459-466, 1976 ; Hoj P.B. *et al.,* FEBS Lett. 230, 67-71, 1988; Hoj P.B. *et al.,* Plant Mol. Biol. 13, 31-42, 1989), bean (Boller T. *et al.,* Planta 157, 22-31, 1983; Broglie K.E. *et al.,* Proc. Natl. Acad. Sci. USA 83, 6820-6824, 1986; Vögeli U. *et al.,* Planta 174, 364-372, 1988); Mauch F. & Staehelin L.A., Plant Cell 1, 447-457, 1989); cucumber (Metraux J.P. & Boller T., Physiol. Mol. Plant Pathol. 28, 161-169, 1986); leek (Spanu P. *et al.,* Planta 177, 447-455, 1989); maize (Nasser W. *et al.,* Plant Mol. Biol. 11, 529-538, 1988), oat (Fink W. *et al.,* Plant Physiol. 88, 270-275, 1988), pea (Mauch F. *et al.,* Plant Physiol. 76, 607-611, 1984; Mauch F. *et al.,* Plant Physiol. 87, 325-333, 1988), poplar (Parsons, T.J. *et al.,* Proc. Natl. Acad. Sci. USA 86, 7895-7899, 1989), potato (Gaynor J.J., Nuc1. Acids Res. 16, 5210, 1988; Kombrink E. *et al.,* Proc. Natl. Acad. Sci. USA 85, 782-786, 1988; Laflamme D. and Roxby R., Plant Mol. Biol. 13, 249-250, 1989), tobacco (e.g. Legrand M. *et al.,* Proc. Natl. Acad. Sci. USA 84, 6750-6754, 1987; Shinshi H. *et al.* Proc. Natl. Acad. Sci. USA 84, 89-93, 1987), tomato (Joosten M.H.A. & De Wit P.J.G.M., Plant Physiol. 89, 945-951, 1989), wheat (Molano J. *et al.,* J. Biol. Chem. 254, 4901-4907, 1979), magainins, lectins, toxins isolated from *Bacillus thuringiensis,* antifungal proteins isolated from *Mirabilis jalapa* (EP 0 576 483) and *Amaranthus* (EP 0 593 501 and US 5,514,779), albumin-type proteins (such as thionine, napin, barley trypsin inhibitor, cereal gliadin and wheat-alpha-amylase, EP 0 602 098), proteins isolated from *Raphanus, Brassica, Sinapis, Arabidopsis, Dahlia, Cnicus, Lathyrus* and *Clitoria* (EP 0 603 216), oxalate oxidase (EP 0 636 181 and EP 0 673 416), saccharide oxidase (PCT/EP 97/04923), antimicrobial proteins isolated from *Allium* seeds and proteins from *Aralia* and *Impatiens* (WO 95/24485) and the like.

Another use of the inducible promoter is to drive proteins which play a role in the gene-for-gene resistance interaction (e.g. as described in WO 91/15585). Such proteins are, for example, plant proteins such as disclosed in Karrer, E.E. *et al.* (Plant Mol. Biol. 36, 681-690, 1998), activated ndr and activated edsl, Cf-proteins, BS3 protein and Pto proteins from tomato, Rpm1 and Rps2 proteins from *Arabidopsis thaliana,* the N-gene from tobacco, the avr-elicitor proteins from *Cladosporium fulvum,* avrBs3 from Xanthomonas, harpins from *Erwinia* and the *avrPto* protein from *Pseudomonas.*

The actual applicability of the invention is not limited to certain plant species, but the plant species to be transformed is preferably from the Cruciferae family. Any plant species that is subject to some form of pathogen attack, may be transformed with chimeric DNA sequences according to the invention, allowing the regulatory region to be induced by pathogen infection thereby triggering production of antipathogenic proteins to be produced in some or all of the plant's cells.

Although some of the embodiments of the invention may not be practicable at present, *e.g.* because some plant species are as yet recalcitrant to genetic transformation, the practising of the invention in such plant species is merely a matter of time and not a matter of principle, because the amenability to genetic transformation as such is of no relevance to the underlying embodiment of the invention.

Transformation of plant species is now routine for an impressive number of plant species, including both the *Dicotyledoneae* as well as the *Monocotyledoneae.* In principle any transformation method may be used to introduce chimeric DNA according to the invention into a suitable ancestor cell, as long as the cells are capable of being regenerated into whole plants. Methods may suitably be selected from the calcium/polyethylene glycol method for protoplasts (Krens, F.A. *et al.,* Nature 296, 72-74, 1982; Negrutiu I. *et al*,, Plant Mol. Biol. 8, 363-373, 1987), electroporation of protoplasts (Shillito R.D. *et al.,* Bio/Technol. 3, 1099-1102, 1985), microinjection into plant material (Crossway A. *et al.,* Mol. Gen. Genet. 202, 179-185, 1986), DNA (or RNA-coated) particle bombardment of various plant material (Klein T.M. *et al.,* Nature 327, 70, 1987), infection with (non-integrative) viruses and the like. A preferred method according to the invention comprises Agrobacterium-mediated DNA transfer. Especially preferred is the use of the so-called binary vector technology as disclosed in EP A 120 516 and U.S. Patent 4,940,838.

Tomato transformation is preferably done essentially as described by Van Roekel et al. (Plant Cell Rep. 12, 644-647, 1993). Potato transformation is preferably done essentially as described by Hoekema et al. (Hoekema, A. *et al.,* Bio/Technology 7, 273-278, 1989).

Generally, after transformation plant cells or cell groupings are selected for the presence of one or more markers which are encoded by plant expressible genes co-transferred with the nucleic acid sequence encoding the protein according to the invention, whereafter the transformed material is regenerated into a whole plant.

Although considered somewhat more recalcitrant towards genetic transformation, monocotyledonous plants are amenable to transformation and fertile transgenic plants can be regenerated from transformed cells or embryos, or other plant material. Presently, preferred methods for transformation of monocots are microprojectile bombardment of embryos, explants or suspension cells, and direct DNA uptake or electroporation (Shimamoto, *et al,* Nature 338, 274-276, 1989). Transgenic maize plants have been obtained by introducing the *Streptomyces hygroscopicus* bar-gene, which encodes phosphinothricin acetyltransferase (an enzyme which inactivates the herbicide phosphinothricin), into embryogenic cells of a maize suspension culture by microprojectile bombardment (Gordon-Kamm,, Plant Cell, 2, 603-618, 1990). The introduction of genetic material into aleurone protoplasts of other monocot crops such as wheat and barley has been reported (Lee, Plant Mol. Biol. 13, 21-30, 1989). Wheat plants have been regenerated from embryogenic suspension culture by selecting only the aged compact and nodular embryogenic callus tissues for the establishment of the embryogenic suspension cultures (Vasil, Bio/Technol. 8, 429-434, 1990). The combination with transformation systems for these crops enables the application of the present invention to monocots.

Monocotyledonous plants, including commercially important crops such as rice and corn are also amenable to DNA transfer by *Agrobacterium* strains *(vide* WO 94/00977; EP 0 159 418 B1; Gould J, *et al.,* Plant. Physiol. 95, 426-434, 1991).

Following DNA transfer and regeneration, putatively transformed plants may be evaluated, for instance using Southern analysis, for the presence of the chimeric DNA according to the invention, copy number and/or genomic organization. In addition, or alternatively, expression levels of the newly introduced DNA may be undertaken, using Northern and/or Western analysis, techniques well known to persons having ordinary skill in the art. After the initial analysis, which is optional, transformed plants showing the desired copy number and expression level of the newly introduced chimeric DNA according to the invention may be tested for resistance levels against pathogens.. Alternatively, the selected plants may be subjected to another round of transformation, for instance to introduce further genes, in order to enhance resistance levels, or broaden the resistance.

Other evaluations may include the testing of pathogen resistance under field conditions, checking fertility, yield, and other characteristics. Such testing is now routinely performed by persons having ordinary skill in the art.

Following such evaluations, the transformed plants may be grown directly, but usually they may be used as parental lines in the breeding of new varieties or in the creation of hybrids and the like.

To obtain transgenic plants capable of constitutively expressing more than one chimeric gene, a number of alternatives are available including the following:
A. The use of DNA, *e.g* a T-DNA on a binary plasmid, with a number of modified genes physically coupled to a selectable marker gene. The advantage of this method is that the chimeric genes are physically coupled and therefore migrate as a single Mendelian locus.
B. Cross-pollination of transgenic plants each already capable of expressing one or more chimeric genes, preferably coupled to a selectable marker gene, with pollen from a transgenic plant which contains one or more chimeric genes coupled to another selectable marker. Afterwards the seed, which is obtained by this crossing, maybe selected on the basis of the presence of the two selectable markers, or on the basis of the presence of the chimeric genes themselves. The plants obtained from the selected seeds can afterwards be used for further crossing. In principle the chimeric genes are not on a single locus and the genes may therefore segregate as independent loci.
C. The use of a number of a plurality chimeric DNA molecules, *e.g.* plasmids, each having one or more chimeric genes and a selectable marker. If the frequency of co-transformation is high, then selection on the basis of only one marker is sufficient. In other cases, the selection on the basis of more than one marker is preferred.
D. Consecutive transformation of transgenic plants already containing a first, second, (etc), chimeric gene with new chimeric DNA, optionally comprising a selectable marker gene. As in method B,the chimeric genes are in principle not on a single locus and the chimeric genes may therefore segregate as independent loci.
E. Combinations of the above mentioned strategies.

The actual strategy may depend on several considerations as maybe easily determined such as the purpose of the parental lines (direct growing, use in a breeding programme, use to produce hybrids) but is not critical with respect to the described invention.

In this context it should be emphasised that plants already containing chimeric DNA may form a suitable genetic background for introducing further chimeric DNAs according to the invention, for instance in order to enhance the production antipathogenic substances, thereby enhancing resistance levels. The cloning of other genes corresponding to proteins that can suitably be used in combination with the regulatory DNA fragments, and the obtention of transgenic plants, capable of relatively over-expressing same, as well as the assessment of their effect on pathogen resistance *in planta,* is now within the scope of the ordinary skilled person in the art.

Plants with improved resistance against pathogens may be grown in the field, in the greenhouse, or at home or elsewhere. Plants or edible parts thereof may be used for animal feed or human consumption, or may be processed for food, feed or other purposes in any form of agriculture or industry. Agriculture shall mean to include horticulture, arboriculture, flower culture, and the like. Industries which may benefit from plant material according to the invention include but are not limited to the pharmaceutical industry, the paper and pulp manufacturing industry, sugar manufacturing industry, feed and food industry, enzyme manufacturers and the like.

The advantages of the plants, or parts thereof, according to the invention are the decreased need for biocide treatment, thus lowering costs of material, labour, and environmental pollution, or prolonging shelf-life of products (e.g. fruit, seed, and the like) of such plants. Plants for the purpose of this invention shall mean multicellular organisms capable of photosynthesis, and subject to some form of pathogen attack. They shall at least include angiosperms as well as gymnosperms, monocotyledonous as well as dicotyledonous plants.

### EXAMPLE 1

### Construction Arabidopsis pMOG553 promotertagging library

The T-DNA sequence of the promoter tagging construct pMOG553 is available in the EMBL database under accession number X84105.

The construct was introduced into *Agrobacterium tumefaciens* strain MOG101 and used for *Arabidopsis thaliana* C24 root transformation. More than 1100 transgenic plants were generated, grown and allowed to self-fertilize and the resulting Sl seeds were harvested.

### EXAMPLE 2

### Infection of Arabidopsis plants with B. cinerea

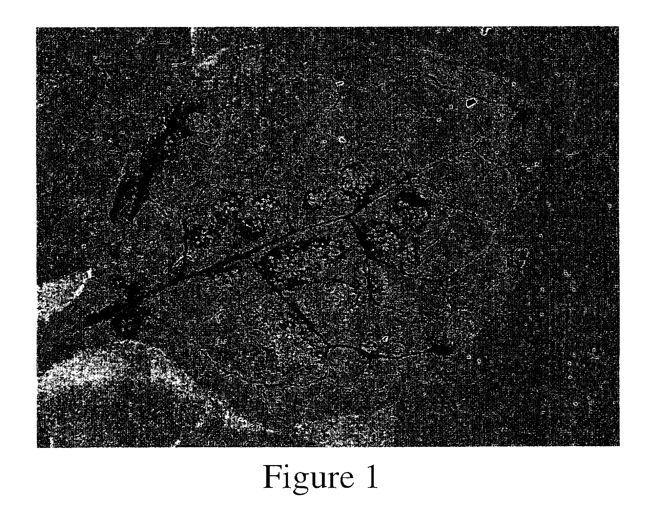

Three weeks old *Arabidopsis* seedlings were transferred to potting soil in 5.5 cm pots and grown for one more week at 18°C. Just before inoculation of the plants leaf samples were taken and a histochemical GUS staining was performed as described in Goddijn *et al.* (The Plant Journal (1993) 4(5): 863-873). The plants were sprayed with a *Botrytis cinerea* spore suspension of 1.2 x 10⁶ spores/ml. After 24 hours and after 48 hours leaves which showed disease symptoms were harvested and again a histochemical GUS staining was performed.
Line number 488 showed expression of the GUS gene just around the *Botrytis* infection site (see Figure 1).

### EXAMPLE 3

### Isolation of Botrytis inducible promoter by inverse PCR.

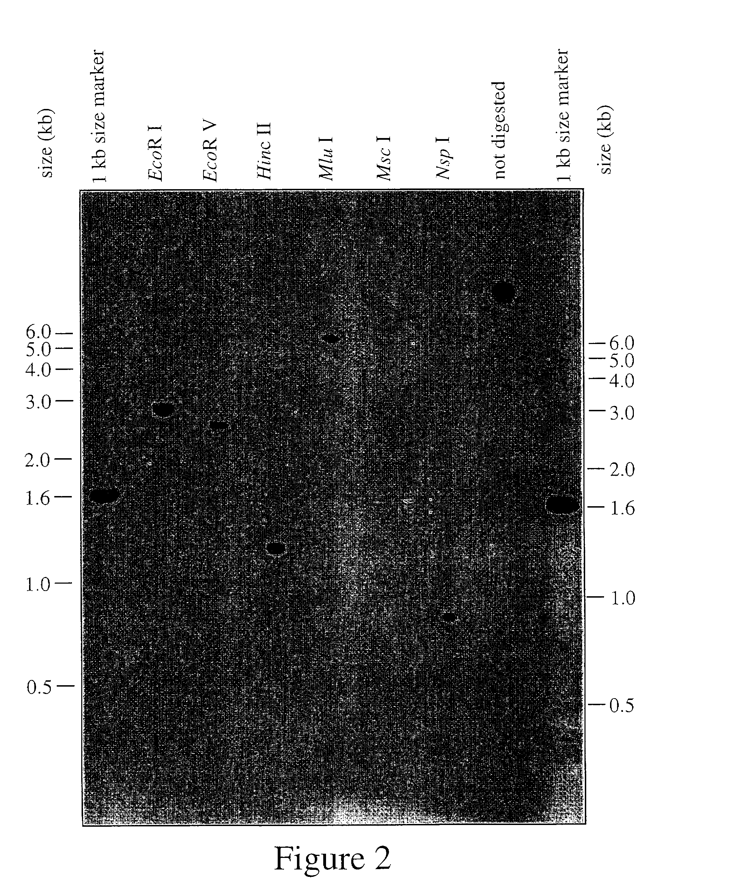

For the isolation of the 5' T-DNA flanking sequence genomic DNA was isolated from leaves of pMOG553 line number 488. The genomic DNA was subjected to restriction enzyme digestion with five different enzymes, *EcoR I, EcoR V, Hinc* II, *Mlu I* and *Nsp I.* For each reaction about 2 µg of DNA was digested in 100 µl water in a 1x concentration of the appropriate reaction buffer and 30 units of restriction enzyme for 16 hours at 37°C. The reaction mixtures were extracted with 1 volume phenol/chloroform/isoamylalcohol (25:24:1, v/v) and precipitated with 2.5 volumes 96% ethanol, washed with 70% ethanol and dissolved in 50 µl water.
25 µl of the mixture was separated on a 0.8% agarose gel in 1x TBE buffer and transferred to a Hybond-N+ (Amersham Life Sciences) membrane using capillary blotting with 0.4 M NaOH. The blot was hybridized (16 hours, 65°C) using a 560 bp GUS fragment (SEQ ID NO: 1; from the *Nco* I site on the ATG start codon until the first *Eco*R V site) labelled with ³²P-dCTP as a probe. Then the blot was washed with a stringency of 0.2x SSC/1% SDS at 65°C. The results of the southern blot (see Figure 2 and table 1) indicated that the T-DNA was only present in one single copy.

**Table 1:**

| Results southern blot and inverse PCR with calculated promoter sizes for each restriction enzyme used. | | | | |
|---|---|---|---|---|
| Restriction enzyme | band size southern blot | on band size iPCR | size sequence | promoter |
| *Eco*R I | 3.0 kb | 1.0 kb | 0.5 kb | |
| *Eco*R V | 2.6 kb | 2.0 kb | 1.8 kb | |
| *Hinc* II | 1.2 kb | 0.6 kb | 0.4 kb | |
| *Mlu* I | 7.0 kb | - | | |
| *Nsp* I | 0.8 kb | 0.2 kb | | |

| | | | | |
|---|---|---|---|---|
| (- means not amplified) | | | | |

The remaining 25 µl was used for ligation in 100 µl lx ligation buffer, 2 units T4 DNA ligase (Gibco BRL) at 14°C for 16 hours. The reaction mixtures were again extracted with phenol/chloroform/isoamylalcohol and the DNA was precipitated with ethanol. The ligated DNA was then linearized with *Sna*B I, extracted with phenol/chloroform/isoamylalcohol, precipitated with ethanol and dissolved in 25 µl water. 5 µl of this DNA sample was used as a template in a PCR reaction with primer GUSINV5: 5'CTT TCC CAC CAA CGC TGA TC3' (SEQ ID NO: 2) in all reactions and as a second primer for the *EcoR* I reaction GUS8 5'CGC ACC ATC GTC GGC TAC AGC3' (SEQ ID NO: 3) and as a second primer for the other reactions GUS7 5'GTA ATG CTC TAC ACC ACG CCG3' (SEQ ID NO: 4). 25 pmol of each primer, 0.5 µl of a 20 mM dNTP solution, 0.5 units Taq DNA polymerase (Gibco BRL) were used to amplify the promoter fragment (1 cycle: 5' 95°C, 5' 55°C, 5' 72°C; 25 cycles: 1' 95°C, 1' 55°C, 2' 72°C; 1 cycle: 1' 95°C, 1' 55°C, 10' 72°C).
The results of the PCR reactions are listed in table 1.

The 0.6 kb *Hinc* II fragment was cut out, purified from the agarose gel and cloned into the T-vector (Promega, Madison WI, USA) as described by the manufacturer. Identity of the cloned PCR fragment was confirmed by DNA sequence analysis.

### EXAMPLE 4

### Isolation of a genomic promoter fragment

For the isolation of a large promoter fragment from the *Arabidopsis* genome a genomic *Arabidopsis thaliana* C24 wildtype library was screened. For the library construction genomic *Arabidopsis* C24 DNA was partially digested with SauIIIa, size selected and cloned into host vector lambda GEM 11. Bacterial host strains used: *E. coli* KW251 and LE392, the average insert size is approximately 17.000 bp, if the complexity of the Arabidopsis genome is estimated to be 190.000 kb, then 54.232 recombinant plaques represent 1 full copy of the *Arabidopsis* genome. The library was amplified using 10 large petridishes containing 80.000 plaques each.

The screening of the genomic library was done using a 401 bp *Hind* III - *Hinc* II fragment (SEQ ID NO: 5) from the T-vector clone harbouring the 0.6 kb PCR fragment (described in Example 3) during the complete procedure.

The genomic library contained 7.5 x 10⁹ pfu/ml. After a first screening round, 42 positive plaques were obtained. Agar plugs of 20 plaques were collected in 1 ml SM buffer and a drop chloroform. Dilutions (10⁻³ and 10⁻⁴ times) were made, combined with *E*. *coli* KW251 cells and plated on agar. From 18 of the 20 plates each 2 agar plugs containing single plaques were removed. Dilutions of 10⁻² and 10⁻⁵ times of each plug eluate were combined with *E*. *coli* KW251 and plated on agar. A plate lysate was prepared from 10 pure plaques. Lambda DNA was isolated from the 10 clones and analysed for their insert by restriction enzyme analysis with *EcoR* I, *EcoR* V and *Sst* I. After separation on a agarose gel the DNA was transferred to a positively charged nylon membrane and hybridized with the 401 bp 488 probe (SEQ ID NO: 5). Hybridizing bands of ± 4 kb (*Sst* I *- Sst* I fragment) of two different subclones were isolated and subcloned into high copy cloning vector pUC18 (Yanisch-Perron, C., Vieira, J. and Messing, J. (1985) Gene 33, 103-119) also digested with *Sst* I. Clones containing inserts of the correct size (4 kb) were selected and subjected to restriction enzyme analysis, southern blotting and hybridization with the 401 bp 488 probe (SEQ ID NO: 5). A 2.3 kb hybridizing *Sst* I *- EcoR* V fragment was subcloned into high copy cloning vector pBKS⁺ (Stratagene) and digested with *Sst* I *- Eco*R V. A clone containing an insert of the correct size was selected and the DNA sequence of the insert was determined (SEQ ID NO: 6). This genomic clone contained the region identical to the 400 bp 488 fragment, an approximately 500 bp downstream region and about 1400 bp upstream promoter sequence. The T-DNA insertion site was probably located at position 1813 in SEQ ID NO: 6. This DNA sequence was used in BLAST homology searches against the EMBL database and EST database where no relevant homologues could be found.

### EXAMPLE 5

### Construction promoter-gus fusions

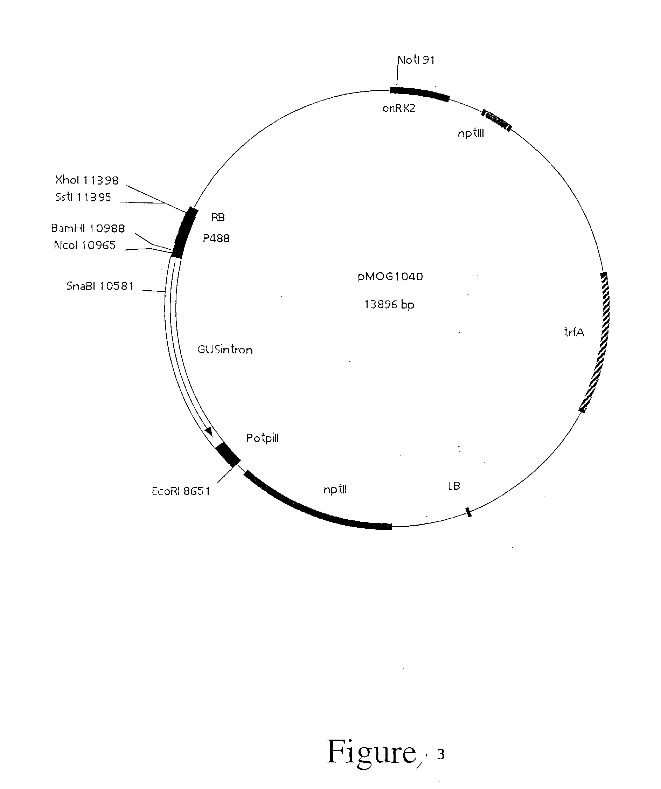

The 400 bp promoter fragment was fused to the *uid*A open reading frame keeping the fusion almost identical as in the original plant line 553-488. One modification was made introducing a *Nco* I restriction site overlapping the ATG startcodon of the *Uid*A open reading frame (ORF). Primers were developed for the amplification of the fragment from the *Arabidopsis* genome: Primer LS259 (5'CGT ACC ATG GGG GAC TGA CC3', SEQ ID NO: 8) introducing a *Nco* I restriction site overlapping the ATG startcodon and primer LS260 (5'AGC CGA GCT CGT TGA CAA AAA AAG TAA AAT AAA GTT C3', SEQ ID NO: 9) introducing a *Sst* I restriction site upstream of the 400 bp promoter fragment. The promoter was amplified using 25 pmol of both primers, 1 µl 20 mM dNTP's and 5 *units pfu* DNA polymerase for 5 cycles at 1' 95°C, 1' 37°C, 2' 72°C; 30 cycles 1' 95°C, 1' 55°C, 2' 72°C and 1 cycle at 1' 95°C, 1' 55°C, 10' 72°C. The obtained PCR product was purified, digested with *Sst* I and *Nco* I and ligated into a *Sst* I, *Nco* I digested cloning vector containing GUSintron (Jefferson *et al*.,(1987) EMBO J 6: 3901-3907) followed by the 3' untranslated region of the potato proteinase inhibitor II gene (Thornburg *et al.,* 1987, Proc. Natl. Acad. Sci. USA 84, 744-748) which contains sequences needed for polyadenylation (An *et al.,* 1989, Plant cell 1, 115-122). The resulting plasmid was named pMOG1039. The complete expression unit was then transferred to binary vector pMOG800 (deposited at the Centraal Bureau voor Schimmelcultures, Baarn, The Netherlands, under CBS 414.93, on august 12, 1993) using restriction enzymes *Sst* I and *Eco*R I. The resulting plasmid was designated pMOG1040 (Figure 3).

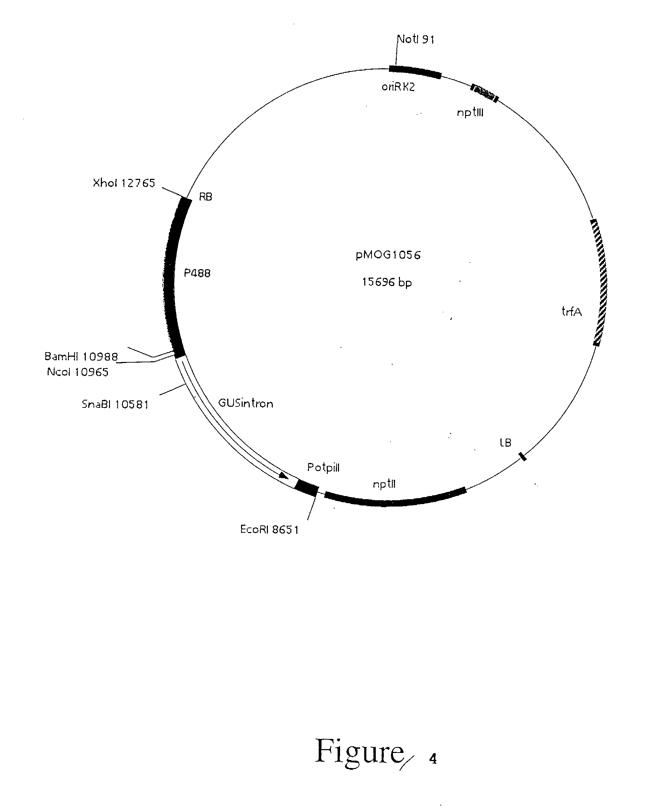

For the construction of the 1800 bp 488 promoter a 1400 bp upstream fragment of the promoter was fused to the 400 bp fragment using the *Hinc* II restriction site. Therefore the 400 bp promoter was excised from vector pMOG1039 with *BamH* I and *Hinc* II and ligated into a likewise digested cloning vector pBKS⁺ (Stratagene). The resulting vector was then digested with *Hinc* II and the 1400 bp upstream *Hinc.*II fragment derived from SEQ ID NO: 6 was ligated into this vector. A clone with the promoter elements fused in a proper way was digested with *Xho* I and *Bam*H I and ligated into binary vector pMOG1040 digested with *Xho* I and *Bam*H I resulting in binary vector pMOG1056 (Figure 4).

Both binary vectors were transferred by electroporation to *Agrobacterium tumefaciens* strain EHA105 for transformation of potato and tomato, strain MOG101 for transformation of tobacco and *Arabidopsis thaliana* and strain MOG301 for transformation *of Brassica napus.*

### EXAMPLE 6

### Transformations of 488 promoter gus constructs to A. thaliana and B. napus Arabidopsis transformation

Described below is the method used for transformation of root segments of *Arabidopsis thaliana* cv. C24 using Agrobacterium tumefaciens. The binary vectors for this transformation method are identical to those described above.
Six mg of *Arabidopsis* seeds were germinated in a flask containing liquid Germination medium under 16 h light period (1700 lux) at 24°C and a 8 h dark period at 21°C at 80 rpm. (The contents of various media can be found in Table X). Roots of 9 days old seedlings were isolated in a sterile petridish and collected in a drop of Germination Medium (GM). Roots were cut in segments of approximately 3-5 mm and approximately 100 explants were spread evenly on a nylon membrane (O 8 cm) which was placed on plates containing Callus Inducing Medium (CIM).The plates were incubated 3 days under the same conditions as described above.

The *Agrobacterium* strain used in this study harboured a rifampicin selection marker in a C 58 chromosomal background. The construction of the helper strain MOG101 is decribed by Hood et al. (1993). Agrobacteria were grown overnight in LB medium with antibiotics (rifampicin 20 mg/l, kanamycin 100 mg/l). The overnight culture was diluted 1:10 in LB without antibiotics and grown for approximately for 3 hours. Bacterial suspensions were centrifuged at 1600xg for 15 minutes at room temperature. Bacteria were resuspended in GM and adjusted to OD600=0.1 and used for cocultivation.

The membrane containing approximately 100 explants was incubated for 2 minutes with the *Agrobacterium* suspension and dried on sterile filter paper to remove excess of bacteria.The membrane with explants are cultured for 48 h on CIM plates. After rinsing the membrane and explants with liquid GM these were incubated on Shoot Induction Medium (SIM) plates containing several concentrations of cyanamide or kanamycin. After 5 days the membrane with the explants was transferred to the same medium (SIM) for subculture. The second subculture was after 2 weeks. Approximately four weeks after cocultivation 60 shoots per cyanamide concentration were excised and placed on plates with Shoot Elongation Medium (SEM) containing 30 mg/l cyanamide. Shoots which were able to root are tested on their transgenic character by testing leaflets and flowers for expression of the gus gene using a histochemical GUS assay.

Transformation *of Brassica napus* wad performed as described in Bade J.B. and Damm, B.: Agrobacterium-mediated transformation of rapeseed. In: Potrykus, I. and Spangeneberg, G. (eds) Gene Transfer to Plants, Springer verlag, heidelberg, 1995, pp. 30-38.

**Table A**

| Media required for Arabidopsis thaliana C24 root transformation | | | | | |
|---|---|---|---|---|---|
| Media components | Media | GM | CIM | SIM | SEM |
| Ingredients | macro elements | B5 | B5 | B5 | MS |
| | micro elements | B5 | B5 | B5 | MS |
| | vitamins | B5 | B5 | B5 | B5 |
| | sucrose (g/l) | | | | 10 |
| | glucose (g/l) | 20 | 20 | 20 | |
| | Daichin agar (g/l) | | 10 | 10 | 10 |
| Hormones | 2,4-D | | 0.5 | | |
| | kinetin | | 0.05 | | |
| | 2-ip | | | 5 | |
| | IAA | | | 0.15 | |
| Antibiotics | vancomycin | | | 100 | 50 |
| | carbenicillin | | | 500 | |
| | cefotaxime | | | | 100 |

### EXAMPLE 7

### Expression profiles of promoter-gus fusions in transgenic plants after fungal infection.

Transgenic *Arabidopsis* plants harbouring the 400 bp promoter gus construct (pMOG1040) and the 1800 bp promoter gus construct (pMOG1056) were infected with *Botrytis cinerea* as described in Example 1. From the pMOG1040 construct 33 plants were tested after infection with *B. cinerea.* None of the transgenic lines with the 400 bp promoter element showed any inducible GUS expression around the fungal infection sites. 14 out of 47 *Arabidopsis* plants harbouring the 1800 bp promoter element (pMOG1056) in front of gus show inducible gus expression after infection with *B. cinerea.*
*Brassica napus* glasshouse plants were infected on detached leaves. Leaves were detached and placed in wet floricultural foam. Small cuttings were made and small droplets of a *B. cinerea* spore suspension were applied on the cuttings allowing the fungus to enter the plant more easily. Fungal infections were allowed to proceed at 18°C and at a high relative humidity (± 90%).

**Table 2:**

| Fungal inducible GUS expression monitored in transgenic *Brassica napus* plants harbouring the pMOG1056 construct after infection with *Botrytis cinerea* (detached leaves). | | |
|---|---|---|
| Plant line number | Expression after *B.cinerea* infection | background expression |
| 1056-1 | + | + |
| 1056-4 | nt | nt |
| 1056-5 | nt | nt |
| 1056-6 | + | - |
| 1056-7 | - | - |
| 1056-8 | - | - |
| 1056-9 | - | + |
| 1056-10 | - | - |
| 1056-11 | - | - |
| 1056-12 | - | - |
| 1056-13 | + | + |
| 1056-14 | - | - |
| 1056-15 | - | + |
| 1056-16 | nt | nt |
| 1056-17 | - | - |
| 1056-18 | - | - |
| 1056-19 | nt | nt |
| 1056-20 | - | + |
| 1056-22 | nt | nt |
| 1056-23 | - | - |
| 1056-24 | nt | nt |
| note: + = GUS expression, - = no detectable GUS expression, | | |
| nt = not tested. | | |

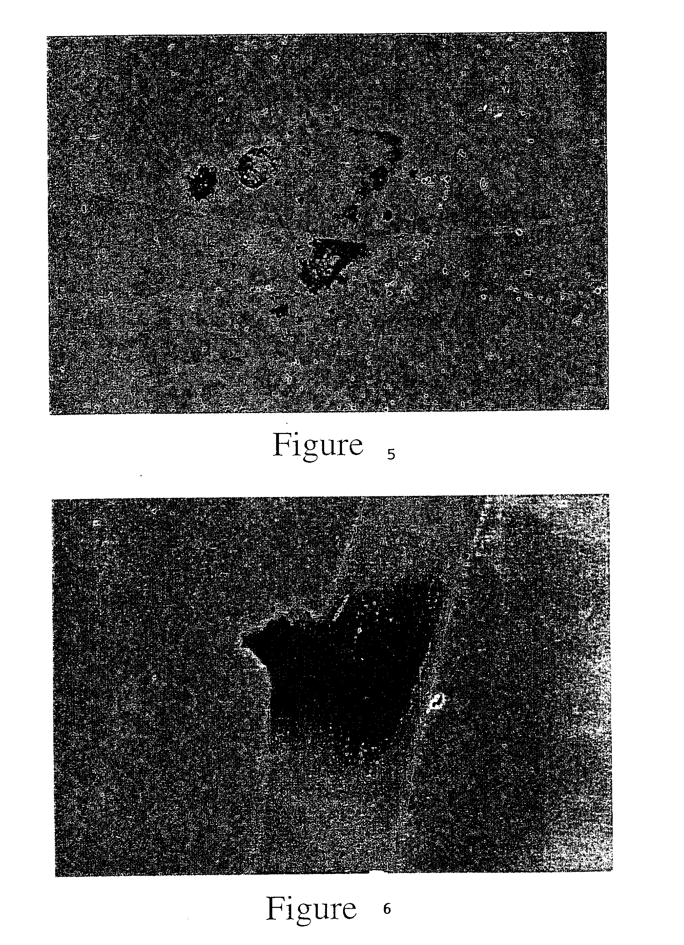

Similar infection assays were performed on *in vitro* plantlets grown in small glass tubes. Plants were inoculated with a spore suspension of the *Brassica napus* pathogen *Phoma lingam.* Small droplets of the spore suspensions were applied directly on the leaves and the disease was allowed to develop for 6 days. When small lesion were visible, leaves were removed from the plant and tested for expression of the GUS gene. About 50% of the transgenic lines show inducible expression of the GUS gene just around the sites of fungal infection (an example is shown in Figure 5). The *Phoma lingam* infection was left to proceed until stem lesions were developing (at approx. 2 weeks after inoculation). Stem segments containing lesions were removed and stained. In several lines expression around the stem lesions was visible (an example is shown in Figure 6).

**Table 3:**

| Fungal inducible GUS expression monitored in transgenic *Brassica napus* plants harbouring the pMOG1056 construct after infection with *Phoma lingam (in vitro)* | | | |
|---|---|---|---|
| Plant line number | *P. lingam* infection | background | Stem lesions |
| 1056-1 | + | - | - |
| 1056-4 | + | - | + |
| 1056-5 | + | - | - |
| 1056-6 | + | - | + |
| 1056-7 | - | - | - |
| 1056-8 | - | - | - |
| 1056-9 | + | - | + |
| 1056-10 | - | - | - |
| 1056-11 | nt | nt | nt |
| 1056-12 | + | - | + |
| 1056-13 | + | - | + |
| 1056-14 | + | - | - |
| 1056-15 | + | - | + |
| 1056-16 | - | - | nt |
| 1056-17 | - | - | nt |
| 1056-18 | - | - | nt |
| 1056-19 | - | - | nt |
| 1056-20 | + | - | nt |
| 1056-22 | - | - | nt |
| 1056-23 | - | - | - |
| 1056-24 | - | - | - |
| note: + = GUS expression,- = no detectable GUS expression, | | | |
| nt = not tested. | | | |

## Claims

1. A DNA fragment obtainable from *Arabidopsis thaliana,* capable of promoting pathogen-inducible transcription of an associated DNA sequence when re-introduced into a plant.

2. A DNA fragment according to claim 1, characterized in that it comprises the nucleotide sequence from 1 to 1728 depicted in SEQ ID NO: 7.

3. A portion or variant of a DNA fragment according to any one of the preceding claims capable of promoting pathogen-inducible transcription of an associated DNA sequence when re-introduced into a plant.

4. A chimeric DNA sequence comprising in the direction of transcription a DNA fragment according to any one of claims 1 to 3 and a DNA sequence to be expressed under the transcriptional control thereof and which is not naturally under transcriptional control of said DNA fragment.

5. A chimeric DNA sequence according to claim 4, wherein the DNA sequence to be expressed causes the production of an antipathogenic protein.

6. A chimeric DNA sequence according to claim 5, wherein said antipathogenic protein is selected from the group consisting of chitinases, glucanases, osmotins, magainins, lectins, saccharide oxidase, oxalate oxidase, oxalate decarboxylase, toxins from *Bacillus thuringiensis,* antifungal proteins isolated from *Mirabilis jalapa, Amaranthus, Raphanus, Brassica, Sinapis, Arabidopsis, Dahlia, Cnicus, Lathyrus, Clitoria, Allium* seeds, *Aralia* and *Impatiens* and albumin-type proteins, such as thionine, napin, barley trypsin inhibitor, cereal gliadin and wheat-alpha-amylase.

7. A chimeric DNA sequence according to claim 4, wherein the DNA sequence to be expressed causes the production of a protein that can induce a hypersensitive response, preferably selected from the group consisting of Cf, Bs3 and Pto proteins from tomato, Rpm1 and Rps2 from *Arabidopsis thaliana,* N-protein from tobacco, avr proteins from *Cladosporium fulvum,* harpins from *Erwinia* and elicitor proteins (avrBs3, avrRpm1, avrRpt2) from *Pseudomonas* or *Xanthomonas.*

8. A replicon comprising a chimeric DNA sequence according to any one of claims 4 to 7.

9. A replicon comprising in the direction of transcription a DNA fragment according to any one of claims 1 to 3 and at least one recognition site for a restriction endonuclease for insertion of a DNA sequence to be expressed under the control of said DNA fragment.

10. A microorganism containing a replicon according to any one of claims 8 or 9.

11. A plant cell having incorporated into its genome a chimeric DNA sequence according to any one of claims 4 to 7.

12. A plant essentially consisting of cells according to claim 11.

13. A plant according to claim 12 which is a dicotyledonous plant.

14. A plant according to any one of claims 11 to 13 belonging to the Cruciferae family.

15. A part of a plant selected from seeds, flowers, tubers, roots, leaves, fruits, pollen and wood, obtained from a plant according to any one of claims 12 to 14.

16. Use of a DNA fragment according to any one of claims 1 to 3 for identifying homologues capable of promoting pathogen-induced transcription in a plant.

17. Use of a chimeric DNA sequence according to any one of claims 4 to 7 for transforming plants.

18. Use of a portion or variant according to claim 3 for making hybrid regulatory DNA sequences.

19. Use of a chimeric DNA sequence according to any of claims 5 to 7 for conferring pathogen resistance to a plant.
